# EUROPÄISCHE PATENTSCHRIFT

(11) **EP 2 544 794 B1**
(45) Veröffentlichungstag und Bekanntmachung des Hinweises auf die Patenterteilung: **16.07.2014**
(21) Anmeldenummer: 11711266.4
(22) Anmeldetag: 08.03.2011
(51) Int. Cl.: B01D 53/26

(54) **VERFAHREN ZUR ADSORPTIVEN TROCKNUNG VON GEREINIGTEM BIOGAS UND REGENERIERUNG BELADENER ADSORPTIONSMITTEL**
METHOD FOR THE ABSORPTIVE DRYING OF PURIFIED BIOGAS AND FOR REGENERATING LADEN ADSORBENTS
PROCÉDÉ DE SÉCHAGE PAR ADSORPTION D'UN BIOGAZ PURIFIÉ ET RÉGÉNÉRATION D'UN AGENT D'ADSORPTION CHARGÉ

(30) Priorität: 12.03.2010 DE 102010011347
(43) Veröffentlichungstag der Anmeldung: 16.01.2013
(73) Patentinhaber: DGE Dr.-Ing. Günther Engineering GmbH, 06886 Wittenberg (DE)
(72) Erfinder: GÜNTHER, Lothar, 82538 Geretsried (DE)
(74) Vertreter: Tragsdorf, Bodo
(86) Internationale Anmeldenummer: PCT/EP2011/001116
(87) Internationale Veröffentlichungsnummer: WO 2011/110322

(56) Entgegenhaltungen:
- EP-A1- 0 207 277
- WO-A1-2008/115079
- WO-A1-2009/101669
- JP-A- 2005 133 939

## Beschreibung

Die Erfindung betrifft ein Verfahren zur adsorptiven Trocknung von gereinigtem Biogas (sogenanntes Biomethan) und Regenerierung beladener Adsorptionsmittel, wobei das getrocknete Biogas einer weiteren Verwendung, z.B. durch Einspeisung in ein herkömmliches Erdgasnetz, zugeführt wird.

Biogas kann nach unterschiedlichen Verfahren gereinigt werden, mit dem Ziel ein Gas mit einem relativ hohen Anteil an Methan, im Bereich von 90 bis 99 Vol.-%, zu erhalten.

Nach der Reinigung kann Biomethan als Restbestandteile noch Kohlendioxid (0,1 bis 4 Vol.-%), Wasserstoff (0,01 bis 0,2 Vol.-%), Stickstoff und Sauerstoff (0,2 bis 4 Vol.-%) und Wasser (1 bis 3 Vol.-%) enthalten. Weitere Komponenten, wie z.B. H₂S, NH₃, sind ggf. noch im Bereich von 1 bis 5 ppm enthalten. In Abhängigkeit vom Vergärungsprozess sind im gereinigten Biogas (Biomethan) noch Anteile an aromatischen oder anderen Kohlenwasserstoffen, in Mengen bis zu 1.000 ppm, enthalten, deren Zündpunkte deutlich unter dem von Methan liegen.

Für eine weitere Verwendung von Biomethan, z.B. zur Einspeisung in ein Erdgasnetz, ist es erforderlich im Gas enthaltenes Wasser bis auf einen Grenzwert von unter 40 mg/Nm³ zu entfernen.

Zur Trocknung bzw. Entfeuchtung von Biogas sind bereits Verfahren zur Kühlung und Kondensation sowie zur Adsorption und Absorption bekannt. Die Kühlung erfolgt meist innerhalb erdverlegter Leitungen, die am tiefsten Punkt mit einem Kondensatabscheider versehen sind. Erforderlichenfalls können zusätzlich noch Wasserkühler eingesetzt werden. Adsorptionsverfahren erfolgen unter Verwendung spezieller Adsorptionsmittel, wie Aktivkohle, Molekularsiebe oder Silicagel. Nach der Beladung können die Adsorbentien durch Erhitzung regeneriert werden.

Absorptive Verfahren (EP 0 907 400 B1) arbeiten mit einem Lösungsmittel, wie Triethylenglykol, wobei durch Gaswäsche im Gas enthaltenes Wasser Triethylenglykol gelöst wird, das anschließend bei einer Temperatur von 200°C wieder regeneriert werden kann.

Diese Verfahrensweise ist sehr energieaufwendig, da im Triethylenglykol gebundenes Wasser verdampft (abdestilliert) werden muss. Da Methan in Triethylenglykol löslich ist (ca. 30g/l bei 1 bar und 25°C) muss mit Methanverlusten gerechnet werden.

In der DE 103 59 959 B4 wird vorgeschlagen, zur Regeneration des feuchten Trocknungsmittels Triethylenglykol ungetrocknetes, feuchtes Biogas zu verwenden.

Aus der DE 20 2008 012 371 U1 ist eine Vorrichtung zur adsorptiven Trocknung von Biogas in einem Festbett (Molekularsiebe) bekannt, wobei Elektroden zum Einsatz kommen, die zur

Erwärmung des Festbettes mit einem HF-Generator zur Einspeisung von HF-Spannung verbunden sind. Die zusätzliche Erwärmung des Feststoffbettes mittels HF-Energie ist mit einem erhöhten Aufwand verbunden. Außerdem besteht ein erhebliches Sicherheitsrisiko, da im Bio- oder Erdgas bestimmte Stoffe enthalten sein können, die zur Selbstentzündung neigen.

Der Erfindung liegt die Aufgabe zugrunde, ein Verfahren zur adsorptiven Trocknung von gereinigtem Biogas mit einem Methangehalt von mindestens 90 Vol.-% und Regenerierung beladener Adsorptionsmittel zu schaffen, bei dem keine Fremdstoffe in das gereinigte Biogas gelangen können, der im zu reinigenden Gas enthaltene Anteil an Methan nahezu unverändert enthalten bleibt, und der Aufwand zur Regeneration des beladenen Adsorptionsmittels verringert wird.

Erfindungsgemäß wird die Aufgabe durch die im Anspruch 1 angegebenen Merkmale gelöst. Vorteilhafte Ausgestaltungen und Weiterbildungen der Verfahrensweise sind Gegenstand der Ansprüche 2 bis 10.

Zur adsorptiven Trocknung von gereinigtem Biogas (Biomethan) werden als Adsorptionsmittel getrennte Schichten auf Basis Silicagel und Molekularsiebe eingesetzt, wobei das zu trocknende Biogas zuerst die Silicagelschicht durchströmt. Vorzugsweise besteht die Schicht aus Silicagel aus zwei Schichten, einer unteren makroporösen und einer oberen mesoporösen Schicht. Das Volumenverhältnis erste Schicht "Silcagel" zu zweite Schicht "Molekularsiebe" beträgt 20:1 bis 3:1, vorzugsweise 12:1 bis 8:1. Dieses ist abhängig vom Wassergehalt des zu trocknenden Biogases (Biomethan). Je höher der Wassergehalt im zu trocknenden Biogas, desto größer ist dieses Verhältnis.

Die Schicht aus Silicagel kann auch aus mindestens zwei Schichten unterschiedlicher Silicagele gebildet werden, wobei der Raum zwischen diesen beiden Schichten über eine externe Wärmequelle erwärmt wird. Dies kann über eine installierte Zwischenheizung erfolgen.

Mit dieser Maßnahme lassen sich Wassergehalte im Biogas von unter 10 mg/Nm³ und Taupunkte von -60 °C bis -80 °C erreichen.

Das zu trocknende Biogas wird bevorzugt drucklos durch den Adsorber gepumpt. Erforderlichenfalls kann auch ein Druck von bis zu 1 bar angewendet werden. Vor der Trocknung des gereinigten Biogases (Biomethan) sollte der Wassergehalt bereits bis auf 5 g/Nm³ reduziert werden. Dies kann beispielsweise durch Kühlung erfolgen. Ohne vorherige Trocknung ist eine entsprechend größere Dimensionierung der Adsorber erforderlich. Die Adsorptionsdauer beträgt 12 bis 14 Stunden. Innerhalb dieser Zeit lässt sich der Wasseranteil im getrockneten Biomethan bis auf deutlich unter 20 mg/Nm³ verringern.

Zur nachfolgenden Regenerierung des Adsorptionsmittels wird ausschließlich erwärmtes getrocknetes Biomethan eingesetzt, das nach dem Kontakt mit Adsorptionsmittel wieder in den Ausgangsstrom an gereinigtem Biogas zurückgeführt wird. Die Erwärmung erfolgt vorzugsweise auf eine Temperatur von ca. 110 bis 150°C in einem separaten Wärmetauscher mittels eines Wärmeträgers, wie Thermalöl oder Warmwasser oder Dampf. Hierzu ist eine exakte Temperaturführung erforderlich, um eine Selbstentzündung von im Biomethan enthaltenen Kohlenwasserstoffen zu vermeiden. Die Temperatur des Wärmeträgers sollte 200°C nicht übersteigen. Während der Regenerierung der Schüttung wird die Temperatur des austretenden Biomethans kontinuierlich gemessen. Diese liegt während der Regeneration in einem Bereich von 20 bis 30°C. Steigt die Austrittstemperatur auf 40 bis 50°C an, so wird die Zufuhr von Biomethan abgeschalten und der Regenerationsprozess beendet.

Vorteilhaft ist es, wenn der Adsorber zu Beginn der Regenerierung mindestens im unteren Bereich erwärmt wird. Dies kann über einen Doppelmantel oder eine Heizschlange erfolgen, die von einem Wärmeträgermedium durchströmt werden. Eine zusätzliche Erwärmung des Adsorbers während der ersten Stunde der Regenerierung ist bereits ausreichend.

Die Dauer der indirekten Beheizung des Adsorbers sollte mindestens 30 Minuten betragen. Das Wärmeträgermedium sollte eine von Temperatur 70 bis 180°C haben.

Die zu beheizende Fläche sollte mindestens 10% der Adsorberhöhe betragen.

Unter diesen Bedingungen zeigte sich, dass bei gleichem Wärmeeintrag die Trocknungszeit für die beladene Schüttung um bis zu 50% reduziert werden kann. Dies wird insbesondere dadurch erreicht, dass die erforderliche Wärme zur Aufheizung der Adsorber schneller als bisher üblich über den Trocknungsgasstrom eingetragen wird. Damit verläuft im Bereich der makroporigen Silicagele, also dort, wo die größte Wassermenge gebunden ist, der Trocknungsprozess wesentlich effektiver. Die indirekte Heizung des Adsorbers während des Trocknungsschrittes ist nur über einen Zeitraum von bis zu 1 Stunde erforderlich. Danach kann die Heizung ausgeschaltet werden.

Für einen unmittelbaren Wiedereinsatz des Adsorbers zur Trocknung von gereinigtem Biogas (Biomethan) ist eine Abkühlung der Schüttung auf Normaltemperatur erforderlich. Hierzu wird gereinigtes Biogas (Biomethan) verwendet, das in einem separaten Wärmetauscher bis auf 5 bis 15°C abgekühlt und durch die Schüttung geleitet wird. Nach ca. zwei Stunden ist die Temperatur der Schüttung wieder im gewünschten Bereich. Das zur Regeneration und Kühlung verwendete Biomethan wird wieder dem Ausgangsstrom an gereinigtem Biogas zugeführt. In den einzelnen Prozessstufen, Vortrocknung und Regenerierung, wird anfallendes Wasser gesammelt und wieder in den Kreislauf zur Erzeugung und/oder Reinigung von Biogas zurückgeführt.

Der Vorteil des vorgeschlagenen Verfahrens besteht in einem geschlossenen Biogas-Stoffkreislauf. Da die Regeneration des Adsorptionsmittels ausschließlich mit gereinigtem Biogas erfolgt, können absolut keine Fremdstoffe während der Trocknung in das Biomethan gelangen. Das getrocknete Biomethan kann somit problemlos, ggf. nach erfolgter Druckerhöhung, in ein Erdgasnetz eingespeist oder einer anderweitigen Verwendung zugeführt werden. Bei einer Rückführung des während der Trocknung anfallenden Kondensates in eine Vorstufe zur Biogaserzeugung entstehen keine Methanverluste. Diese Verfahrensweise ist somit sehr wirtschaftlich. Die gewählte Kombination der Zusammensetzung der Adsorberschüttung ist in ihrer Aktivität auf die abzutrennende Wassermenge und die Regenerationsbedingungen abgestimmt. Der Wassergehalt im gereinigten Biogas (Biomethan) lässt sich somit von ca. 5 g/Nm³ bis auf mindestens 10 mg/Nm³ oder noch darunter verringern, wobei die Zeitdauer bis zur maximalen Beladung und die Zeitdauer für eine thermisch schonende Regeneration (Temperatur max. bis 150°C) und Kühlung der beladenen Schüttung in etwa gleich gehalten werden können. Unter thermisch günstigeren Bedingungen, indirekte Beheizung des Adsorbers, kann die Regenerationsdauer für die beladenen Adsorbersäule noch weiter verringert werden.

Der während der Beladung stattfindende Massetransfer, die Übertragung des Wassers vom Biogas auf die Adsorbentien, verschiebt sich, bis das Ende der Adsorbersäule erreicht ist und es zum Durchbruch des feuchten Biogases kommt, was jedoch in der Praxis durch eine vorzeitige Beendigung ausgeschlossen wird. Durch die am Ende angeordnete Schüttung Molekularsiebe verlängert sich die wirksame Massetransferzone. Die Wasseraufnahme erfolgt zuerst über das Slicagel bis zum Gleichgewicht. Von der nachfolgenden Schicht Molekularsiebe wird Wasser zwar langsamer aufgenommen, aber die Beladungskapazität erhöht sich insgesamt und ermöglicht das Erreichen der geringen Restgehalte an Wasser von ca. 10 mg/Nm³. Bei Erreichen dieses Wertes wird die Trocknung beendet, ohne die Obergrenze der Aufnahmekapazität zu erreichen. Dadurch wird eine ausreichende Regenerierung der Molekularsiebe bei vergleichweise niedrigen Temperaturen (max. bis 150°C) erst möglich. Höhere Regenerationstemperaturen würden aufgrund der Selbstentzündungsgefahr den Einsatz von Biomethan verbieten.

Durch Anlegen eines Unterdruckes während der Regeneration kann die Entfernung von Wasser verbessert werden und es kann gegebenenfalls mit geringeren Regenerationstemperaturen gearbeitet werden.

Die Erfindung wird nachstehend an zwei Beispielen erläutert. In der zugehörigen Zeichnung ist das Funktionsschaltbild einer Anlage zur Durchführung des Verfahrens dargestellt.

### Beispiel 1

A: Trocknung:
Aus biologischem Abfallmaterial in einem Fermenter erzeugtes Biogas wird von unerwünschten Nebenbestandteilen gereinigt, wobei enthaltenes Kohlendioxid mittels einer drucklosen Aminwäsche entfernt wird. Aus der Waschkolonne werden 30 Nm³/h an gereinigtem Biogas, sogenanntem Biomethan mit einer Temperatur von 38°C abgezogen. Das Biomethan hat folgende Zusammensetzung:

| | |
|---|---|
| CH₄ | 95,2 Vol.-% |
| CO₂ | 0,5 Vol.-% |
| H₂O | 4,0 Vol.-% |
| O₂ | 0,1 Vol.-% |
| N₂ | 0,2 Vol.-% |
| H₂S | 2 ppm |
| NH₃ | 1 ppm |

Das über die Leitung 1 abgezogene Biomethan wird in einem ersten Wärmetauscher W 1 auf ca. 22°C und anschließend über die Leitung 2 zum nachgeschalteten, zweiten Wärmetauscher W2 geleitet und in diesem bis auf 5°C abgekühlt. Dadurch wird der Wasseranteil im Biomethan von ursprünglich 19.500 mg/Nm³ bis auf 5.035 mg/Nm³ reduziert. Über die Leitungen 9 und 10 wird abgeschiedenes Wasser in einen nicht näher gezeigten Behälter abgeführt, gegebenenfalls zwischengelagert und wieder der Biogaserzeugung oder -reinigung zugeführt.

Die Menge an Biomethan verringert sich auf 28,99 Nm³/h. Das über die Leitung 3 abgeführte Biomethan hat sich in seiner Zusammensetzung wie folgt verändert:

| | |
|---|---|
| CH₄ | 98,52 Vol.-% |
| CO₂ | 0,52 Vol.-% |
| H₂O | 0,66 Vol.-% |

Zur Trocknung des Biomethans sind nachfolgend zwei identische Adsorber A1 und A2 mit einer Schütthöhe von 700 mm und einem Durchmesser von 300 mm angeordnet, die abwechselnd zur Trocknung des Biomethans eingesetzt und nach erfolgter Beladung einer Regeneration unterzogen werden. Während sich der eine Absorber im Trocknungsbetrieb befindet wird der andere Adsorber regeneriert.

Beide Adsorber A 1 und A2 enthalten eine lose Schüttung, bestehend aus zwei unterschiedlichen Adsorbentien S1 und S2. Die untere Schüttung S1 besteht aus 20 l Silicagel mit einem mittleren Porendurchmesser von >50 bis 100 nm (Makroporen) und 30 l Silicagel mit einem mittleren Porendurchmesser von 25 nm (Mesoporen), die durch einen gasdurchlässigen Boden getrennt sind. Die Öffnungen im Boden sind so klein, dass ein Vermischen unterschiedlicher Silicagelpartikel ausgeschlossen ist.

Oberhalb der mesoporösen Silicagelschüttung ist eine Schüttung S2 aus Molekularsieben (Zeolithen) mit einem Porendurchmesser von 0,4 nm angeordnet, in einer Menge von 5 l. Die Silicagelschüttung und die Molekularsiebe sind durch einen gasdurchlässigen Boden getrennt, sodass sich die Partikel nicht vermischen können. Das Verhältnis von Schüttung S1:Schüttung S2 beträgt 50:5 = 10.

Im kontinuierlichen Betrieb wird das Biomethan über die Leitung 3 einem der beiden Adsorber A1 oder A2 zugeführt, z.B. dem Adsorber A1, und während des Kontaktes mit den Adsorbentien getrocknet. Gleichzeitig, also parallel, wird der andere Adsorber A2, der zwischenzeitlich beladen wurde, regeneriert.

Das zu trocknende Biomethan strömt nach dem Verlassen des zweiten Wärmetauschers W2 mit einer Temperatur von 5°C von unten durch die Schüttung in der Adsorbersäule A1 und wird am Kopf des Adsorbers A1 über die Leitung 4 abgezogen. Die Trocknung erfolgt an sich drucklos, es liegt nur der Saugdruck zur Förderung des Gasstromes an, der ca. 10 mbar beträgt.

Entsprechend dem konkreten Anwendungsfall werden die Dimensionierung der Adsorbersäule und die Strömungsgeschwindigkeit experimentell ermittelt, unter Berücksichtigung einer möglichst effektiven Massetransferzone.

Die spezielle Auswahl und Zusammensetzung der Adsorptionsmittel ermöglicht eine relativ lange Nutzungsdauer. Erst nach einer Adsorptionsdauer von 14 Stunden kommt es zum Durchbruch des feuchten Gases mit der Folge der Umleitung des Gasstromes auf die andere, zwischenzeitlich regenerierte Adsorbersäule 2. Während der ersten 14 Stunden der Adsorption beträgt der Wasseranteil im getrockneten Biomethan nur noch 10 mg/Nm³ (Taupunkt -63°C). Das über die Leitung 5 nach erfolgter Verdichtung abgezogene getrocknete Biomethan (Temperatur ca. 25°C) kann direkt in ein Erdgasnetz eingespeist werden. Würde man die Adsorptionsdauer um ca. 1 Stunde, auf 15 Stunden, verlängern, so erhöht sich der der Wasseranteil im getrockneten Biomethan bis auf 153 mg/Nm³ (Taupunkt -40°C).

### B: Regeneration

Während der in der einen Adsorbersäule A1 stattfindenden Trocknung des Biomethans wird die beladene Adsorbersäule A2 wie folgt regeneriert:

In die Leitung 4 zur Abführung des getrockneten Biomethans ist nach dem Verdichter V1 eine absperrbare Abzweigleitung 6 eingebunden, über die 5 m³/h getrocknetes Biomethan ausgekreist werden. Dieser Teilstrom wird durch einen dritten Wärmetauscher W3 geleitet und bis auf eine Temperatur von ca. 120°C erwärmt und über die Leitung 7 zu dem zu regenerierenden Adsorber geleitet. Der heiße Teilstrom getrocknetes Biomethan wird zur Regeneration der Adsorberpartikel von oben durch die beladene Adsorbersäule A2 geleitet und nimmt dabei unter Abkühlung die in diesen Partikeln enthaltene Feuchtigkeit auf. Der für die Regenerierung bestimmte Teilstrom wird mit einem Druck von 40 mbar durch die Adsorbersäule gepumpt.

Nach einer Regerationsdauer von ca. 12 Stunden besitzt die Adsorberschüttung wieder eine ausreichende Beladungskapazität. Das mit Feuchtigkeit beladene Biomethan wird im Kreislauf wieder dem zu trocknenden Biomethangasstrom zugeführt.

Für einen unmittelbaren Wiedereinsatz der Adsorberschüttung zur Trocknung ist jedoch noch eine Abkühlung der Adsorbentien erforderlich. Hierzu wird das über die Leitung 6 ausgekreiste getrocknete Biomethan nicht erwärmt sondern direkt über einen vierten Wärmetauscher W4 geleitet und in diesem bis auf ca. 10°C abgekühlt und durch die Schüttung im Adsorber A2 geleitet. Der Transport des abgekühlten Biomethans erfolgt über die Leitung 7. Das zur Abkühlung verwendete Biomethan wird über die Leitung 11 wieder in den Biomethankreislauf zurückgeführt. Nach einer Kühldauer von 2 Stunden erfolgt die Umschaltung von Adsober A1 zu Adsorber A2. Nunmehr wird der beladene Adsorber A1 regeneriert und der Adsorber A2 zur Trocknung eingesetzt.

Bezogen auf die Einsatzmenge an zu trocknendem Biomethan (ca. 30 Nm³/h) werden über die Leitungen 9 und 10 1,2 l/h an Wasser mit einem Methangehalt an 28 g/l ausgetragen. Das Wasser kann gesammelt und dann direkt wieder der Biogasreinigung als Waschwasser zugeführt werden. Die im Kondensat enthaltene Methanmenge beträgt 0,034 kg/h bzw. 0,024 Nm³/h. Dies entspricht einem Anteil von 0,084 % der zu trocknenden Methanmenge. Der Vorteil dieser Verfahrensweise ist, dass keine Methanverluste auftreten.

Bei Anlagen mit einer Trocknung von ca. 5.000 Nm³/h Biomethan würde es ansonsten zu Methanverlusten in Höhe 4 Nm³/h kommen.

### Beispiel 2

Die Regeneration der beladenen Adsorbersäule wird unter gleichen Bedingungen wie im Beispiel 1 durchgeführt, wobei zusätzlich während der ersten Stunde der untere Abschnitt (bis zu einer Höhe von ca. 300 mm) der Adsorbersäule mit einem flüssigen Wärmeträger mit einer Temperatur von 150°C erwärmt wird. Gleichzeitig werden wie im Beispiel 1 5 m³/h getrocknetes Biomethan mit einer Temperatur von ca. 120°C und einem Druck von 40 mbar durch die Schüttung der Adsorbersäule geleitet.

Durch die indirekte zusätzliche Erwärmung wird der Trocknungsprozess für die SilicagelSchüttung beschleunigt, die den größeren Anteil an Wasser bzw. Feuchtigkeit enthält.

Da durch die Erwärmung der Adsorbersäule bereits ein Teil der im Silicagel enthaltenen Feuchtigkeit ausgetrieben wird, kann die im Trocknungsgas (Biomethan) enthaltene Wärme somit effektiver für die weitere Trocknung der Schüttung genutzt werden, da der Adsorber durch die indirekte Erwärmung bereits in dem Bereich, wo sich der größte Anteil an Feuchtigkeit befindet, aufgeheizt wird.

Nach einer Trocknungsdauer von ca. 8 Stunden hat die Adsorberschüttung wieder eine ausreichende Beladungskapazität erreicht und wird analog wie im Beispiel noch zwei Stunden gekühlt. Nach 10 Stunden Regeneration ist die Adsorberschüttung wieder voll einsatzfähig. Beim nachträglichen Einsatz der Adsorbersäule zeigte sich, dass die Schüttung im Vergleich zu Beispiel 1 eine deutlich höhere Beladungskapazität besitzt.

Während der ersten 14 Stunden der Adsorption des zu trocknenden Biogases beträgt der Wasseranteil im getrockneten Biomethan nur noch 4 mg/Nm³ (Taupunkt -70°C). Bei einer Verlängerung der Adsorptionsdauer um ca. 1 Stunde, auf 15 Stunden, erhöht sich der Wasseranteil im getrockneten Biomethan bis auf 10 mg/Nm³ (Taupunkt -63°C).

Dieses Beispiel zeigt, dass bei Anwendung einer indirekten Adsorberbeheizung eine deutlich bessere Trocknung mit geringeren Wassergehalten und kürzerer Trocknungszeit erreicht werden kann.

### Beispiel 3

Bei gleichen Bedingungen, wie im Beispiel 2, wird jetzt die Regenerationstemperatur von 120 auf 140°C erhöht.

Unter diesen Bedingungen beträgt der Wasseranteil im getrockneten Biomethan bis zu 10 Stunden 50 bis 80 mg/Nm³, danach steigt dieser in 1 Stunde auf 160 mg/Nm³.

Damit wird gezeigt, dass trotz höherem Energieaufwand von Beispiel 2 nur eine geringe Verbesserung der Trocknung erreicht werden kann. Bei der Verwendung höherer Regenerationstemperaturen kommt es zur Schädigung der Adsorberschüttung. Eine Erhöhung der Trocknungsgasmenge über Leitung 4 von 5 auf 10 Nm³/h verringert den Wassergehalt im getrockneten Biomethan während der ersten 10 Stunden auf 40 bis 70 mg/Nm³, erfordert aber die doppelte Regenerationsleistung.

### Vergleichsbeispiel 1

Im Unterschied zu Beispiel 1 wird zur Trocknung des feuchten Biomethans nur eine Schüttung, bestehend aus 55 l Silicagel mit einem Porendurchmesser von 25 nm, verwendet. Unter gleichen Bedingungen wie im Beispiel 1 kommt es nach einer Adsorptionsdauer von 10 Stunden bereits zum Durchbruch des feuchten Gases.

Im getrockneten Biomethan wurde während der Adsorptionsdauer ein Restgehalt an Wasser von 100 bis 120 mg/Nm³ ermittelt.

### Vergleichsbeispiel 2

Bei gleichen Bedingungen, wie im Beispiel 2, werden jetzt als Trocknungsmittel 20 l Silicagel mit einem Porendurchmesser von 50-100 nm und darüber nur 35 l Silicagel mit einem Porendurchmesser von 25 nm verwendet.

Unter diesen Bedingungen beträgt der Wasseranteil im getrockneten Biomethan bis zu 12 Stunden 40 bis 50 mg/Nm³, danach steigt dieser in 1 Stunde auf 145 mg/Nm³.

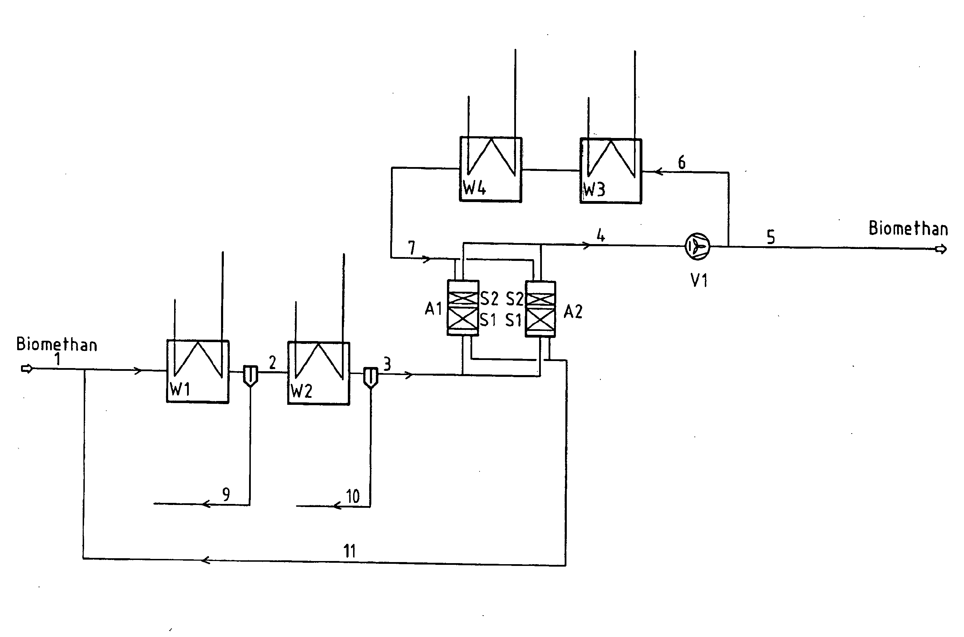

## Patentansprüche

1. Verfahren zur adsorptiven Trocknung von gereinigtem Biogas (Biomethan) mit einem Methangehalt von mindestens 90 Vol.-% und Regenerierung beladener Adsorptionsmittel unter Verwendung von mindestens zwei Adsorbern, die feste Adsorptionsmittel als lose Schüttung enthalten, die abwechselnd beladen und mittels eines erwärmten Gasstromes regeneriert werden, **dadurch gekennzeichnet, dass** Trocknung und Regeneration als geschlossener Biogas-Kreislauf geführt werden, wobei als Adsorptionsmittel getrennte Schichten auf Basis Silicagel und Molekularsiebe eingesetzt werden, das zu trocknende Biogas zuerst die Silicagelschicht durchströmt, und zur Regenerierung des Adsorptionsmittels ausschließlich erwärmtes getrocknetes Biomethan mit einer Temperatur von bis zu 150°C eingesetzt wird, das nach dem Kontakt mit Adsorptionsmittel wieder in den Ausgangsstrom an gereinigtem Biogas zurückgeführt wird und nach erfolgter Regenerierung gekühltes, getrocknetes Biomethan mit dem Adsorptionsmittel in Kontakt gebracht wird, das anschließend wieder in den Ausgangsstrom an gereinigtem Biogas zurückgeführt wird, und während der Trocknung und Regenerierung anfallendes methanhaltiges Wasser wieder der Biogaserzeugung und/oder -reinigung zugeführt wird.

2. Verfahren nach Anspruch 1, **dadurch gekennzeichnet, dass** aus dem gereinigten Biogas (Biomethan) vor der Zuführung zum Adsorber eine Teilmenge an Wasser durch Kühlung entfernt wird.

3. Verfahren nach einem der Ansprüche 1 oder 2, **dadurch gekennzeichnet, dass** die Schicht aus Silicagel aus mindestens zwei Schichten, einer unteren makroporösen und einer oberen mesoporösen Schicht gebildet wird, in einem Verhältnis von unterer zu oberer Schicht von 1:1 bis 1:4.

4. Verfahren nach einem der Ansprüche 1 bis 3, **dadurch gekennzeichnet, dass** die Schicht aus Silicagel aus mindestens zwei Schichten unterschiedlicher Silicagele gebildet wird, wobei der Raum zwischen diesen beiden Schichten über eine externe Wärmequelle erwärmt wird.

5. Verfahren nach einem der Ansprüche 1 bis 4, **dadurch gekennzeichnet, dass** das zur Regenerierung eingesetzte Biomethan mit einer Temperatur von 110 bis 150°C durch die Schüttung geleitet wird, wobei kontinuierlich die Temperatur des austretenden Biomethans gemessen wird und nach einem Anstieg der Austrittstemperatur auf 40 bis 50°C die Zufuhr von Biomethan abgeschalten wird.

6. Verfahren nach einem der Ansprüche 1 bis 5, **dadurch gekennzeichnet, dass** das zu trocknende Biogas drucklos oder mit geringem Überdruck bis zu 1 bar durch den Adsorber gepumpt wird.

7. Verfahren nach einem der Ansprüche 1 bis 6, **dadurch gekennzeichnet, dass** das Volumenverhältnis erste Schicht (Silicagel) zu zweite Schicht (Molekularsiebe) 20:1 bis 3:1 beträgt.

8. Verfahren nach einem der Ansprüche 1 bis 7, **dadurch gekennzeichnet, dass** die Adsorptionsdauer 12 bis 14 Stunden beträgt und der Wasseranteil im getrockneten Biomethan bis auf unter 20 mg/Nm³ verringert wird.

9. Verfahren nach einem der Ansprüche 1 bis 8, **dadurch gekennzeichnet, dass** die regenerierten Adsorbentien vor einer Wiederverwendung mit gekühltem Biomethan (Temperatur 5 bis 15°C) bis auf 10 bis 30°C abgekühlt werden.

10. Verfahren nach einem der Ansprüche 1 bis 9, **dadurch gekennzeichnet, dass** während der Regeneration mindestens der untere Bereich des Adsorbers indirekt erwärmt wird.

11. Verfahren nach einem der Ansprüche 1 bis 10, **dadurch gekennzeichnet, dass** die Regeneration des beladenen Adsorbers bei Unterdruck erfolgt.

## Claims

1. A process for adsorptive drying of purified biogas (biomethane) having a methane content of at least 90% by volume and regeneration of laden adsorbents using at least two adsorbers which comprise solid adsorbents as a loose bed which are alternately laden and regenerated by means of a heated gas stream, **characterized in that** drying and regeneration are conducted as a closed biogas circuit, and the adsorbents used are separate layers based on silica gel and molecular sieves, the biogas to be dried flows first through the silica gel layer, and the adsorbent is regenerated using exclusively heated dried biomethane having a temperature of up to 150°C which, after contact with adsorbent, is recycled back into the starting stream of purified biogas and, on completion of regeneration, cooled dried biomethane is contacted with the adsorbent and is then recycled back into the starting stream of purified biogas, and methane-containing water obtained during the drying and regeneration is sent back to the biogas production and/or purification.

2. The process as claimed in claim 1, **characterized in that** a portion of water is removed by cooling from the purified biogas (biomethane) prior to supply to the adsorber.

3. The process as claimed in either of claims 1 and 2, **characterized in that** the layer of silica gel is formed from at least two layers, a lower macroporous layer and an upper mesoporous layer, in a ratio of lower to upper layer of 1:1 to 1:4.

4. The process as claimed in any of claims 1 to 3, **characterized in that** the layer of silica gel is formed from at least two layers of different silica gels, the space between these two layers being heated by means of an external heat source.

5. The process as claimed in any of claims 1 to 4, **characterized in that** the biomethane used for regeneration is passed through the bed at a temperature of 110 to 150°C, with continuous measurement of the temperature of the exiting biomethane and shutdown of the supply of biomethane after a rise in the exit temperature to 40 to 50°C.

6. The process as claimed in any of claims 1 to 5, **characterized in that** the biogas to be dried is pumped through the adsorber at ambient pressure or with a slightly elevated pressure of up to 1 bar.

7. The process as claimed in any of claims 1 to 6, **characterized in that** the volume ratio of first layer (silica gel) to second layer (molecular sieves) is 20:1 to 3:1.

8. The process as claimed in any of claims 1 to 7, **characterized in that** the adsorption time is 12 to 14 hours and the water content in the dried biomethane is reduced down to below 20 mg/m³ (STP).

9. The process as claimed in any of claims 1 to 8, **characterized in that** the regenerated adsorbents, prior to reuse, are cooled down to 10 to 30°C with cooled biomethane (temperature 5 to 15°C).

10. The process as claimed in any of claims 1 to 9, **characterized in that** at least the lower region of the adsorber is heated indirectly during the regeneration.

11. The process as claimed in any of claims 1 to 10, **characterized in that** the laden adsorber is regenerated under reduced pressure.

## Revendications

1. Procédé pour le séchage par adsorption de biogaz purifié (biométhane) contenant au moins 90 % de méthane et pour la régénération d'agents adsorbants chargés en utilisant au moins deux adsorbants contenant des agents adsorbants solides sous forme de garnissage en vrac, qui sont en alternance chargés et régénérés au moyen d'un courant gazeux réchauffé, **caractérisé en ce que** le séchage et la régénération sont réalisés sous forme de circuit de biogaz fermé, des couches séparées à base de gel de silice et de tamis moléculaires étant utilisées comme agent adsorbant, le biogaz à sécher parcourant d'abord la couche de gel de silice, et pour la régénération de l'agent adsorbant on utilise exclusivement du biométhane séché et réchauffé, présentant une température inférieure ou égale à 150°C, qui est ramené, au contact de l'agent adsorbant, dans le courant initial de biogaz purifié et, au terme de la régénération, le biométhane séché et refroidi est mis au contact de l'agent adsorbant qui finit par être ramené dans le courant initial de biogaz purifié, et pendant le séchage et la régénération, l'eau produite contenant du méthane est ramenée à la production et/ou la purification de biogaz.

2. Procédé selon la revendication 1, **caractérisé en ce qu'**une quantité partielle d'eau est enlevée, en étant refroidie, du biogaz purifié (biométhane) avant l'ajout à l'adsorbant.

3. Procédé selon l'une quelconque des revendications 1 ou 2, **caractérisé en ce que** la couche de gel de silice est formée d'au moins deux couches, une couche inférieure macroporeuse et une couche supérieure mésoporeuse, le rapport de la couche inférieure par rapport à la couche supérieure étant compris entre 1:1 et 1:4.

4. Procédé selon l'une quelconque des revendications 1 à 3, **caractérisé en ce que** la couche de gel de silice est formée d'au moins deux couches de gels de silice différents, l'espace entre les deux couches étant réchauffé par une source de chaleur externe.

5. Procédé selon l'une quelconque des revendications 1 à 4, **caractérisé en ce que** le biométhane utilisé pour la régénération et présentant une température comprise entre 110°C et 150°C parcourt le garnissage, la température du biométhane sortant étant mesurée en continu et l'ajout de biométhane étant arrêté une fois que la température de sortie a atteint 40 à 50°C.

6. Procédé selon l'une quelconque des revendications 1 à 5, **caractérisé en ce que** le biogaz à sécher est pompé par l'adsorbant sans pression ou à une faible surpression allant jusqu'à 1 bar.

7. Procédé selon l'une quelconque des revendications 1 à 6, **caractérisé en ce que** le volume de la première couche (gel de silice) par rapport à celui de la seconde couche (tamis moléculaires) est compris entre 20:1 et 3:1.

8. Procédé selon l'une quelconque des revendications 1 à 7, **caractérisé en ce que** la durée d'adsorption est comprise entre 12 et 14 heures et la part d'eau contenue dans le biométhane séché est abaissée à moins de 20 mg/Nm³.

9. Procédé selon l'une quelconque des revendications 1 à 8, **caractérisé en ce qu'**avant d'être réutilisés, les adsorbants régénérés sont refroidis à l'aide de biométhane refroidi (température comprise entre 5 et 15°C) à une température comprise entre 10 et 30°C.

10. Procédé selon l'une quelconque des revendications 1 à 9, **caractérisé en ce que**, pendant la régénération, au moins la zone inférieure de l'adsorbant est réchauffée de manière indirecte.

11. Procédé selon l'une quelconque des revendications 1 à 10, **caractérisé en ce que** la régénération de l'adsorbant chargé s'effectue sous dépression.
